# EUROPÄISCHE PATENTSCHRIFT

(11) **EP 4 222 138 B1**
(45) Veröffentlichungstag und Bekanntmachung des Hinweises auf die Patenterteilung: **06.11.2024**
(21) Anmeldenummer: 21798278.4
(22) Anmeldetag: 29.09.2021
(51) Int. Cl.: C07C 213/06, B60C 1/00, C08L 21/00, C07C 215/82

(54) **VERBINDUNG, KAUTSCHUKMISCHUNG ENTHALTEND DIE VERBINDUNG, FAHRZEUGREIFEN, DER DIE KAUTSCHUKMISCHUNG IN WENIGSTENS EINEM BAUTEIL AUFWEIST, VERFAHREN ZUR HERSTELLUNG DER VERBINDUNG SOWIE VERWENDUNG DER VERBINDUNG ALS ALTERUNGSSCHUTZMITTEL UND/ODER ANTIOXIDATIONSMITTEL UND/ODER ANTIOZONANT UND/ODER FARBSTOFF**
COMPOUND, RUBBER MIXTURE CONTAINING THE COMPOUND, VEHICLE TIRE WHICH HAS AT LEAST ONE COMPONENT COMPRISING THE RUBBER MIXTURE, METHOD FOR PRODUCING THE COMPOUND, AND USE OF THE COMPOUND AS AN AGING PROTECTION AGENT AND/OR ANTIOXIDANT AGENT AND/OR ANTIOZONANT AND/OR DYE
COMPOSÉ, MÉLANGE DE CAOUTCHOUC CONTENANT LE COMPOSÉ, PNEU DE VÉHICULE QUI COMPREND AU MOINS UN COMPOSANT COMPRENANT LE MÉLANGE DE CAOUTCHOUC, PROCÉDÉ DE PRODUCTION DU COMPOSÉ, ET UTILISATION DU COMPOSÉ EN TANT QU'AGENT DE PROTECTION CONTRE LE VIEILLISSEMENT ET/OU AGENT ANTIOXYDANT ET/OU ANTI-OZONANT ET/OU COLORANT

(30) Priorität: 02.10.2020 DE 102020212507
(43) Veröffentlichungstag der Anmeldung: 09.08.2023
(73) Patentinhaber: Continental Reifen Deutschland GmbH, 30175 Hannover (DE)
(72) Erfinder: JACOB, Andreas, 30165 Hannover (DE); DAUER, David-Raphael, 30165 Hannover (DE); STROHMEIER, Julian, 30165 Hannover (DE); DREIER, Anna-Lena, 30165 Hannover (DE); RECKER, Carla, 30165 Hannover (DE); BECKER, Jörg-August, 30167 Hannover (DE); MATZ, Florian, 30167 Hannover (DE); GRAF, Rebecca, 30167 Hannover (DE); FLORMANN, Jan, 30167 Hannover (DE)
(74) Vertreter: Continental Corporation
(86) Internationale Anmeldenummer: PCT/DE2021/200132
(87) Internationale Veröffentlichungsnummer: WO 2022/069002

(56) Entgegenhaltungen:
- EP-A1- 0 617 004
- US-B1- 6 770 785

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zur Herstellung der Verbindung sowie die Verwendung der Verbindung als Alterungsschutzmittel und/oder Antioxidationsmittel und/oder Antiozonant und/oder Farbstoff.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials, verkürzt oder es kommt zur nachträglichen Härtung des Werkstoffes.

Alterungsschutzmittel tragen daher maßgeblich zur Langlebigkeit von Fahrzeugreifen und anderen technischen Gummiartikeln bei.

Bekannte Alterungsschutzmittel sind aromatische Amine, wie beispielsweise 6-PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl-, Alkoxy- und Alkylperoxyradikalen, reagieren und diese somit abfangen und somit die Kautschuke etc. vor weiteren Oxidationsreaktionen schützen.

Alterungsschutzmittel, die insbesondere mit Ozon reagieren und dieses abfangen, werden auch als "Ozonschutzmittel" oder "Antiozonant" bezeichnet.

EP 0 617 004 A1 offenbart beispielsweise ein Verfahren zur Herstellung von N-substituiertem N'-phenyl-p-phenylendiaminen, insbesondere zur Verwendung als Antioxidationsmittel bzw. Antiozonant.

Der Erfindung liegt die Aufgabe zugrunde, eine neuartige Verbindung bereitzustellen, die insbesondere als Alterungsschutzmittel in Fahrzeugreifen oder anderen technischen Gummiartikeln verwendet werden kann, um somit auf Basis des Standes der Technik eine verbesserte Schutzwirkung dieser Artikel zu erzielen.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist. Ferner wird die Aufgabe durch die Verfahren zur Herstellung der Verbindung sowie die Verwendung der Verbindung als Alterungsschutzmittel und/oder Antioxidationsmittel und/oder Antiozonant gelöst.

Die Verbindung gemäß Anspruch 1 kann ferner als Farbstoff verwendet werden.

Die Verbindung gemäß Anspruch 1 weist die Formel I) auf:

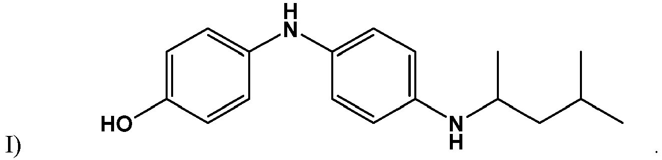

Es handelt sich bei der Verbindung gemäß Formel I) somit um N-(1,3-Dimethylbutyl)-N'-p-hydroxyphenyl-p-phenylendiamin.

Die Verbindung gemäß Formel I) weist gegenüber den bekannten Alterungsschutzmitteln 6PPD oder IPPD vermutlich aufgrund der Hydroxygruppe eine erhöhte Reaktivität gegenüber Sauerstoff, Ozon oder Radikalen (s. oben) auf, wodurch mit der Verbindung gemäß Formel I) eine verbesserte Schutzwirkung, insbesondere in Fahrzeugreifen und anderen technischen Gummiartikeln, aber auch in Ölen und Schmierstoffen, erzielt wird. Die Erfindung soll aber nicht an einen bestimmten Wirkmechanismus oder eine bestimmte Erklärung gebunden sein.

Die DE1220124 offenbart eine Verbindung gemäß Formel S1):

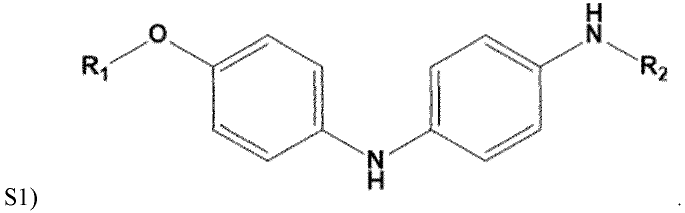

Gegenüber der Verbindung gemäß Formel S1) weist die erfindungsgemäße Verbindung gemäß Formel I) mit der Hydroxygruppe einen weiteren Wasserstoffdonor auf, wodurch die Verbindung gemäß Formel I) mehr Radikale abfangen kann.

Kombinationen aus verschiedenen Alterungsschutzklassen, z.B. Phenole mit aromatischen Aminen, sind zudem aus der CN 105272892 und CN 107935867 bekannt. Allerdings stehen die einzelnen Bausteine nicht in direkter Konjugation, sondern sind immer durch einen aliphatischen Spacer isoliert. Es ergibt sich also keine direkte Beeinflussung bzw. Wechselwirkung zwischen den Gruppen. Die erfindungsgemäße Verbindung gemäß Formel I) weist somit gegenüber den Verbindungen aus der CN 105272892 und CN 107935867 ebenfalls eine verbesserte Reaktivität und damit Schutzwirkung auf.

Die US 6770785B1 offenbart die Verbindungen gemäß Formel S2 und S3.

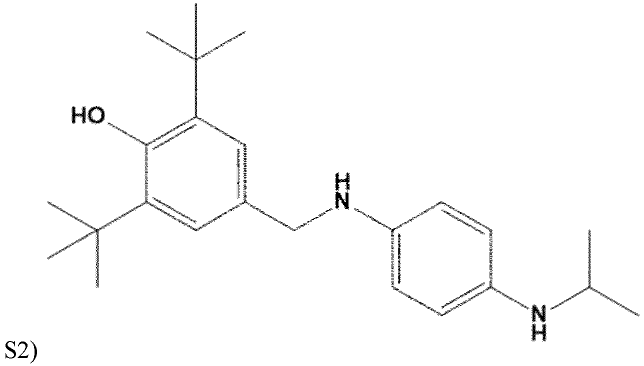

Gegenüber der Verbindung gemäß Formel S2) weist die erfindungsgemäße Verbindung gemäß Formel I) den Vorteil auf, dass deren Bausteine in Konjugation stehen, wodurch sich eine bessere Schutzwirkung ergibt. In Formel S2) ist die Konjugation durch die -CH₂-NH-Gruppierung unterbrochen.

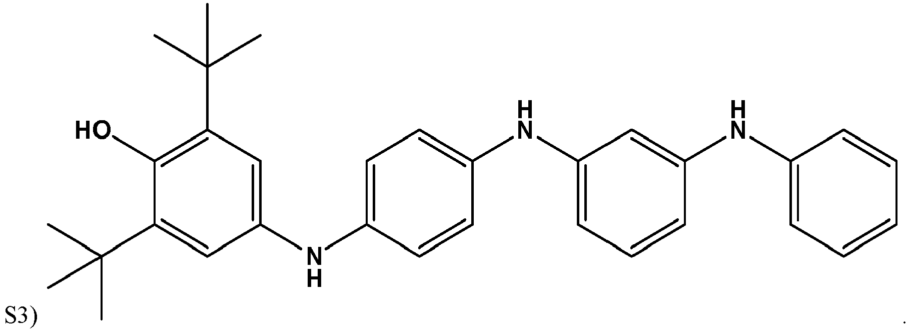

Gegenüber der Verbindung gemäß Formel S3) weist die erfindungsgemäße Verbindung gemäß Formel I) den Vorteil auf, dass sich am äußeren Stickstoffatom eine aliphatische und keine aromatische Gruppe befindet, wodurch sich eine bessere Schutzwirkung gegenüber Ozon ergibt.

Verbindungen bzw. Moleküle, die am äußeren Stickstoffatom statt der 1,3-Dimethylbutyl-Gruppe und einem Wasserstoffatom nur aliphatische oder aromatische Reste aufweisen, also kein Wasserstoffatom, und damit am Stickstoffatom maximal substituiert sind, weisen im Vergleich zu der erfindungsgemäßen Verbindung gemäß Formel I) ebenfalls einen schlechteren Schutz gegenüber Ozon auf, da dieser Teil des Moleküls die Reaktivität maßgeblich beeinflusst und die Möglichkeit der Abspaltung eines Wasserstoffatoms vom Stickstoffatom die Schutzwirkung mit verursacht.

Die erfindungsgemäße Verbindung gemäß Formel I) ist besonders als Alterungsschutzmittel und/oder Ozonschutzmittel in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Die erfindungsgemäße Verbindung gemäß Formel I) ist besonders zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon geeignet.

Zu Verwendung der Verbindung gemäß Formel I) in den genannten Artikeln oder Stoffen wird diese in einer Zusammensetzung verwendet und in dieser eingemischt verwendet. Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) in Ölen, Schmierstoffen, wie insbesondere Treib- oder Betriebsstoffen für Motoren. Insbesondere kann die erfindungsgemäße Verbindung somit in Motoren verwendet werden.

Ein weiterer Gegenstand der Erfindung ist die Verwendung der erfindungsgemäßen Verbindung gemäß Formel I) als Farbstoff in Fasern und/oder Polymeren und/oder Papier und/oder in (Streich-)Farben und Lacken.

Ein weiterer Gegenstand der Erfindung ist somit eine Kautschukmischung.

Die erfindungsgemäße Kautschukmischung enthält die Verbindung gemäß Formel I). Bei der erfindungsgemäßen Kautschukmischung kann es sich prinzipiell um jede Kautschukmischung handeln, in der die neuartige erfindungsgemäße Verbindung gemäß Formel I) verbesserte Eigenschaften, insbesondere eine erhöhte Langlebigkeit durch Alterungsschutz und/oder Ozonschutz erzielt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 5 phr, ganz besonders bevorzugt 1 bis 5 phr, der Verbindung gemäß Formel I).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (Mw größer als 20.000 g/mol) und dadurch festen Kautschuke bezogen.

Gemäß vorteilhafter Ausführungsformen der Erfindung enthält die erfindungsgemäße Kautschukmischung wenigstens einen Dienkautschuk.

Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, der fachkundigen Person bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt eine gute Verarbeitbarkeit und Reversionsstabilität sowie optimierte Reißeigenschaften und ein optimales Rollwiderstandsverhalten.

Gemäß einer besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Kieselsäuren.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr. In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Bei der Kieselsäure kann es sich um die dem Fachmann bekannten Kieselsäuretypen, die als Füllstoff für Reifenkautschukmischungen geeignet sind, handeln. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Für den Fall, dass wenigstens zwei verschiedene Kieselsäuren, die sich z. B. durch ihre BET-Oberfläche unterscheiden, in der erfindungsgemäßem Kautschukmischung enthalten sind, beziehen sich die genannten Mengenangaben auf die Gesamtmenge aller enthaltenen Kieselsäuren.

Die Begriffe "Kieselsäure" und "Silika" werden im Rahmen der vorliegenden Erfindung synonym verwendet.

Die Kautschukmischung kann zudem weitere Füllstoffe enthalten, wie insbesondere Ruße, insbesondere Industrieruße oder Pyrolyse-Ruße, oder weitere Füllstoffe, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filier".

Zinkoxid gehört im Rahmen der vorliegenden Erfindung nicht zu den Füllstoffen.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Als Ruße kommen alle der fachkundigen Person bekannten Rußtypen in Frage.

In einer Ausführungsform hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 80 bis 150 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst.

Bevorzugt ist hierbei, wenn lediglich ein Rußtyp in der jeweiligen Kautschukmischung verwendet wird, es können aber auch verschiedene Rußtypen in die Kautschukmischung eingemischt werden.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel, wie z. B. Diamine, wie N-Phenyl-N'-(1,3-dimethylbutyl)-p-phenylendiamin (6PPD), N,N`-Diphenyl-p-phenylendiamin (DPPD), N,N'-Ditolyl-p-phenylendiamin (DTPD), N-(1,4-dimethylpentyl)-N'-phenyl-p-phenylendiamin (7PPD), N-Isopropyl-N'-phenyl-p-phenylendiamin (IPPD), oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Kieselsäure),
d) Ozonschutzwachse,
e) Harze, insbesondere Klebharze für innere Reifenbauteile,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts) und/oder RAE (Residual Aromatic Extract) und/oder TDAE (Treated Destillated Aromatic Extracts) und/oder MES (Mild Extracted Solvents) und/oder naphthenische Öle.

Gemäß besonders vorteilhafter Ausführungsformen enthält die erfindungsgemäße Kautschukmischung neben den erfindungsgemäßen gemäß Formel I) keine weiteren Alterungsschutzmittel aus der Gruppe der p-Phenylendiamine, s. obige Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von Diaminen, die ausgewählt sind aus der Gruppe enthaltend, bevorzugt bestehend aus, N-Phenyl-N'-(1,3-dimethylbutyl)-p-phenylendiamin (6PPD), N,N`-Diphenyl-p-phenylendiamin (DPPD), N,N'-Ditolyl-p-phenylendiamin (DTPD), N-Isopropyl-N'-phenyl-p-phenylendiamin (IPPD), N-(1,4-dimethylpentyl)-N'-phenyl-p-phenylendiamin (7PPD).

Mit den bevorzugt sehr geringen Mengen von 0 bis 0 phr bzw. besonders bevorzugt 0 phr an den genannten Diaminen und der erfindungsgemäß enthaltenen Verbindung gemäß Formel I) ist es möglich eine verbesserte Schutzwirkung zu erzielen. Hierbei ersetzt die erfindungsgemäße Verbindung gemäß Formel I) die genannten im Stand der Technik bekannten Phenylendiamine.

Gemäß weiterer vorteilhafter Ausführungsformen der Erfindung ist noch wenigstens ein weiteres der genannten Diamin-Alterungsschutzmittel enthalten, sodass die erfindungsgemäße Verbindung die im Stand der Technik bekannten Diamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Gemäß weiterer vorteilhafter Ausführungsformen enthält die erfindungsgemäße Kautschukmischung keine weiteren Alterungsschutzmittel, d. h. 0 phr an weiteren Alterungsschutzmitteln außer der erfindungsgemäßen Verbindung gemäß Formel I).

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen der Kieselsäure oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln:
-SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3 `-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern und/oder Mercaptobeschleunigern und/oder Sulfenamidbeschleunigern und/oder Thiocarbamatbeschleunigern und/oder Thiurambeschleunigern und/oder Thiophosphatbeschleunigern und/oder Thioharnstoffbeschleunigern und/oder Xanthogenat-Beschleunigern und/oder Guanidin-Beschleunigern.

Bevorzugt ist die Verwendung eines Sulfenamidbeschleunigers, der ausgewählt ist aus der Gruppe bestehend aus N-Cyclohexyl-2-benzothiazolsufenamid (CBS) und/oder N,N-Dicyclohexylbenzothiazol-2-sulfenamid (DCBS) und/oder Benzothiazyl-2-sulfenmorpholid (MBS) und/oder N-tert-Butyl-2-benzothiazylsulfenamid (TBBS) oder eines Guanidin-Beschleunigers wie Diphenylguanidin (DPG).

Als schwefelspendende Substanz können dabei alle dem Fachmann bekannten schwefelspendenden Substanzen verwendet werden. Enthält die Kautschukmischung eine schwefelspendende Substanz, ist diese bevorzugt ausgewählt aus der Gruppe enthaltend z.B. Thiuramdisulfide, wie z.B. Tetrabenzylthiuramdisulfid (TBzTD) und/oder Tetramethylthiuramdisulfid (TMTD) und/oder Tetraethylthiuramdisulfid (TETD), und/oder Thiuramtetrasulfide, wie z.B. Dipentamethylenthiuramtetrasulfid (DPTT), und/oder Dithiophosphate, wie z.B.

DipDis (Bis-(Diisopropyl)thiophosphoryldisulfid) und/oder Bis(O,O-2-ethylhexyl-thiophosphoryl)Polysulfid (z. B. Rhenocure SDT 50^{®}, Rheinchemie GmbH) und/oder Zinkdichloryldithiophosphat (z. B. Rhenocure ZDT/S^{®}, Rheinchemie GmbH) und/oder Zinkalkyldithiophosphat, und/oder 1,6-Bis(N,N-dibenzylthiocarbamoyldithio)hexan und/oder Diarylpolysulfide und/oder Dialkylpolysulfide.

Auch weitere netzwerkbildende Systeme, wie sie beispielsweise unter den Handelsnamen Vulkuren^{®}, Duralink^{®} oder Perkaiink^{®} erhältlich sind, oder netzwerkbildende Systeme, wie sie in der WO 2010/049216 A2 beschrieben sind, können in der Kautschukmischung eingesetzt werden. Dieses System enthält ein Vulkanisationsmittel, welches mit einer Funktionalität größer vier vernetzt und zumindest einen Vulkanisationsbeschleuniger.

Besonders bevorzugt ist die Verwendung der Beschleuniger TBBS und/oder CBS und/oder Diphenylguanidin (DPG).

Außerdem können in der Kautschukmischung Vulkanisationsverzögerer vorhanden sein.

Die Begriffe "vulkanisiert" und "vernetzt" werden im Rahmen der vorliegenden Erfindung synonym verwendet.

Gemäß einer bevorzugten Weiterbildung der Erfindung werden bei der Herstellung der schwefelvernetzbaren Kautschukmischung mehrere Beschleuniger in der Fertigmischstufe zugegeben.

Die Herstellung der erfindungsgemäßen schwefelvernetzbaren Kautschukmischung erfolgt nach dem in der Kautschukindustrie üblichen Verfahren, bei dem zunächst in ein oder mehreren Mischstufen eine Grundmischung mit allen Bestandteilen außer dem Vulkanisationssystem (Schwefel und vulkanisationsbeeinflussende Substanzen) hergestellt wird. Durch Zugabe des Vulkanisationssystems in einer letzten Mischstufe wird die Fertigmischung erzeugt. Die Fertigmischung wird z.B. durch einen Extrusionsvorgang oder Kalandrieren weiterverarbeitet und in die entsprechende Form gebracht. Anschließend erfolgt die Weiterverarbeitung durch Vulkanisation, wobei aufgrund des im Rahmen der vorliegenden Erfindung zugegebenen Vulkanisationssystems eine Schwefelvernetzung stattfindet.

Die oben beschriebene erfindungsgemäße Kautschukmischung ist besonders für die Verwendung in Fahrzeugreifen, insbesondere Fahrzeugluftreifen geeignet.

Zur Verwendung in Fahrzeugreifen wird die Mischung als Fertigmischung vor der Vulkanisation bevorzugt in die Form eines Laufstreifens gebracht und bei der Herstellung des Fahrzeugreifenrohlings wie bekannt aufgebracht.

Die Herstellung der erfindungsgemäßen Kautschukmischung zur Verwendung als Seitenwand oder sonstige Body- Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage. Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen oder vorliegen können.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I) somit auch in mehreren Bauteilen in ggf. angepasster Zusammensetzung aufweisen.

Ein weiterer Gegenstand der vorliegenden Erfindung ist ein Verfahren zur Herstellung der Verbindung gemäß Formel I), wobei das Verfahren wenigstens die folgenden Verfahrensschritte umfasst:
a) Bereitstellung der Substanz gemäß Formel A) 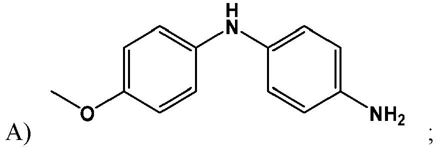
b) Bereitstellung von Methylisobutylketon (MIBK) und Wasserstoff oder von Methylisobutylketon (MIBK) und Ameisensäure ;
c) Umsetzung der Substanz gemäß Schritt a) mit den Substanzen gemäß Schritt b), bevorzugt in Gegenwart eines Hydrierungskatalysators, zu der Substanz gemäß Formel C) 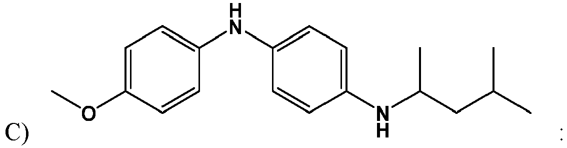
d) Bereitstellung einer Lewis-Säure, wie insbesondere Bortribromid (BBr₃), Aluminiumtrichlorid (AlCl₃), Aluminiumtribromid (AlBr₃), Bortrifluoriddiethyletherat (BF₃*OEt₂) oder Zinntetrachlorid (SnCl₄), oder einer Brönsted-Säure (Brønsted-Säure), wie insbesondere Iodwasserstoff (HI) oder Bromwasserstoff (HBr);
e) Umsetzung der Substanz gemäß Formel C) mit der Substanz aus Schritt d) zu der Substanz gemäß Formel I)

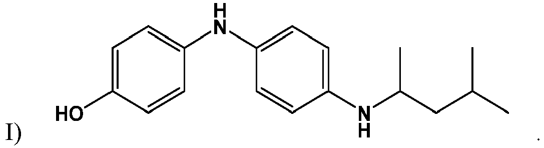

Bevorzugt ist in Schritt b) die Alternative Methylisobutylketon (MIBK) und Wasserstoff gegenüber MIBK und Ameisensäure.

Bevorzugt wird die in Schritt c) enthaltene Substanz gemäß Formel C) in einem organischen Lösungsmittel, bevorzugt einem aprotischen Lösungsmittel, wie insbesondere Dichlormethan (DCM) oder Chloroform (CHCl₃), gelöst und dann gemäß Schritt e) umgesetzt.

Ein weiterer Gegenstand der vorliegenden Erfindung ist ein weiteres Verfahren zur Herstellung der Verbindung gemäß Formel I), wobei das Verfahren wenigstens die folgenden Verfahrensschritte umfasst:
a1) Bereitstellung der Substanz gemäß Formel A) 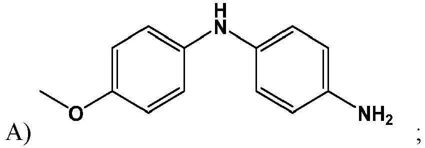
   b1) Bereitstellung einer Lewis-Säure, wie insbesondere Bortribromid (BBr₃), Aluminiumtrichlorid (AlCl₃), Aluminiumtribromid (AlBr₃), Bortrifluoriddiethyletherat (BF₃*OEt₂) oder Zinntetrachlorid (SnCl₄), oder einer Brönsted-Säure (Brønsted-Säure), wie insbesondere Iodwasserstoff (HI) oder Bromwasserstoff (HBr);
   c1) Umsetzung der Substanz gemäß Schritt a1) mit der Substanz aus Schritt
b1) zu der Substanz gemäß Formel C1) 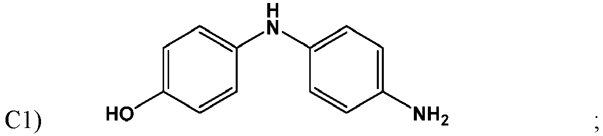
   d1) Bereitstellung von Methylisobutylketon (MIBK) und Wasserstoff;
   e1) Umsetzung der Substanz gemäß Formel C1) mit den Substanzen
      aus Schritt d1), bevorzugt in Gegenwart eines Hydrierungskatalysators, zu der Substanz gemäß Formel I)

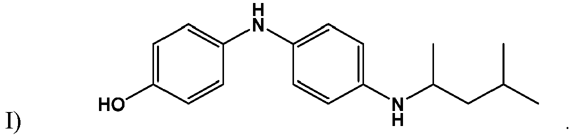

Bevorzugt wird in den Verfahrensschritten, in denen eine Umsetzung mit Wasserstoff erfolgt, ein geeigneter Katalysator, im Rahmen der vorliegenden Erfindung als "Hydrierungskatalysator" bezeichnet, verwendet.

Bevorzugt ist der Hydrierungskatalysator der Verfahren (Schritt c) bzw. e1)) ein Edelmetallkatalysator, wie insbesondere Palladium (Pd) oder Platin (Pt). Bevorzugt wird das Edelmetall auf Kohle (C) eingesetzt, wie Palladium auf Kohle (Pd/C).

Ferner können auch andere bekannte Katalysatoren, wie Raney-Nickel oder Kupferchromit verwendet werden.

Bevorzugt wird in Schritt c) Palladium auf Kohle (Pd/C) als Hydrierungskatalysator verwendet.

Bevorzugt wird in Schritt e1) Palladium auf Kohle (Pd/C) als Hydrierungskatalysator verwendet.

Der Wasserstoffdruck bei den jeweiligen Verfahrensschritten, bei denen Wasserstoff verwendet wird, beträgt bevorzugt 1 bis 40 bar, besonders bevorzugt 10 bis 30 bar, ganz besonders bevorzugt 15 bis 25 bar.

Die Hydrierungsreaktion in Schritt c) bzw. e1) findet bevorzugt in einem Autoklaven, insbesondere einem Edelstahlautoklaven, statt.

Die Temperatur in Verfahrensschritt c) sowie e1) beträgt bevorzugt von Raumtemperatur (RT, insbesondere 20 °C) bis 100 °C, besonders bevorzugt 40 bis 80 °C, ganz besonders bevorzugt 40 bis 70 °C.

Bei zu hohen Temperaturen kommt es zu einer unerwünschten Hydrierung der aromatischen Bestandteile.

Sämtliche Verfahrensschritte, insbesondere Schritt e) und c1), finden vorteilhafterweise unter Schutzgasatmosphäre statt.

Die Verfahrensschritte e) und c1) finden bevorzugt bei einer Temperatur von -78 (minus achtundsiebzig) °C bis +20 °C, besonders bevorzugt -10 °C bis +10 °C, insbesondere bei 0°C, statt.

Die Reaktionen in den Verfahrensschritten e) bzw. c1) stellen jeweils eine Etherspaltung dar. Prinzipiell ist hierzu jegliche Substanz geeignet, mit der die erwünschte Etherspaltung der Substanz C) bzw. A) durchgeführt werden kann. Die Substanz, mit der die Etherspaltung durchgeführt wird, wird im Rahmen der vorliegenden Erfindung auch als "Etherspaltreagenz" bezeichnet.

Hierzu kann eine Lewis-Säure, wie insbesondere Bortribromid (BBr₃), Aluminiumtrichlorid (AlCl₃), Aluminiumtribromid (AlBr₃), Bortrifluoriddiethyletherat (BF₃*OEt₂) oder Zinntetrachlorid (SnCl₄), oder eine Brönsted-Säure (Brønsted-Säure), wie insbesondere Iodwasserstoff (HI) oder Bromwasserstoff (HBr), verwendet werden. Brönsted-Säuren (Brønsted-Säuren) sind dem Fachmann bekannt und können über den pKa-Wert, auch pKs-Wert, charakterisiert weren.

Starke Brönsted-Säuren weisen einen pKa-Wert von -1,74 (minus einskommasiebenvier) bis 4,5 (vierkommafünf) und sehr starke Säuren einen von weniger als -1,74 auf, wie sich aus dem Römpp Online Lexikon (URL: https://roempp.thieme.de/lexicon/RD-19-00110?searchterm=s%C3%A4ure) ergibt.

Im Rahmen der vorliegenden Erfindung sind sehr starke Brönstedt-Säuren mit einem pKa-Wert von weniger als -1,74 bevorzugt, wie insbesondere Iodwasserstoff (HI) oder Bromwasserstoff (HBr).

Gemäß besonders vorteilhafter Ausführungsformen der Erfindung wird in c1) und e) eine Lewis-Säure, insbesondere Bortribromid (BBr₃), verwendet.

Die Substanz gemäß Formel A) sowie Bortribromid (BBr3) und Methylisobutylketon (MIBK) sind kommerziell erhältlich. Auch die anderen genannten Alternativen zu BBr3 sind kommerziell erhältlich.

Im Folgenden soll die Erfindung anhand von Ausführungsbeispielen näher erläutert werden.

Die Verbindung gemäß Formel I) wurde auf folgende Weisen hergestellt:

### X1): Synthese von N-(L3-Dimethylbutyl)-N' -p-methoxyphenyl-p-phenylendiamin (Verbindung gemäß Formel C)) gemäß Schritt c):

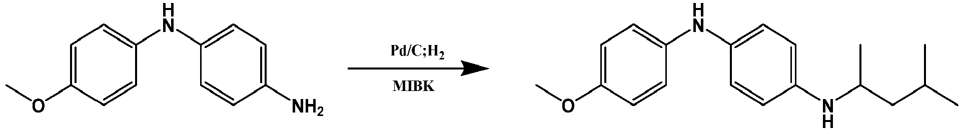

In einen Edelstahlautoklaven, der mit einem Tefloninliner bestückt wurde, wurden 5.00 g (23.3 mmol, 1 Äq) 4-(4-Methoxyphenylamino)anilin, 2.00 g Pd/C (Palladium auf Kohle C) (5%) (0.2 g auf 4.67 mmol Substrat) und 50.0 mL Methylisobutylketon (MIBK) eingewogen. Anschließend wurde 20 bar Wasserstoff (H₂) aufgedrückt und bei 60°C für 10 Stunden rühren gelassen. Nach Beendigung der Reaktion wurde der überschüssige Wasserstoff abgeblasen und die Suspension über Kieselgur (Celite^{®}) filtriert sowie mit Ethanol nachgewaschen. Das Filtrat wurde bis zur Trockne eingeengt und im Vakuum getrocknet. Der Rückstand wurde an Kieselgel aufgereinigt (Cyclohexan/Essigester 100:0 → 80:20). Oranges Öl; Ausbeute 5.6 g (80 % d. Th.).

¹H-NMR (*engl.* "nuclear magnetic resonance") (500 MHz, DMSO-*d6*) δ = 8.09 (s, 1H), 6.94 (td, *J* = 7.6, 1.5 Hz, 1H), 6.88 (dd, *J* = 7.6, 1.4 Hz, 1H), 6.69 - 6.63 (m, 2H), 6.51 (d, *J* = 8.4 Hz, 1H), 6.27 (dd, *J* = 8.5, 2.5 Hz, 1H), 6.21 (d, *J* = 2.5 Hz, 1H), 4.82 (d, *J* = 8.9 Hz, 1H), 3.30 (dt, *J* = 8.6, 6.5 Hz, 1H), 1.70 (dp, *J* = 13.5, 6.7 Hz, 1H), 1.38 (dt, *J* = 13.9, 7.1 Hz, 1H), 1.16 (dt, *J* = 13.5, 6.8 Hz, 1H), 1.02 (d, *J* = 6.1 Hz, 3H), 0.87 (dd, *J* = 19.4, 6.6 Hz, 6H).

¹³C-NMR (126 MHz, DMSO-*d6*) δ = 151.8, 143.0, 140.0, 133.0, 120.7, 116.0, 114.5, 113.6, 55.2, 46.1, 45.9, 26.3, 24.5, 22.8, 22.6, 20.87 (46.1 und 45.9 ist die Aufspaltung als Folge von Enantiomeren).

ESI-MS (Elektrosprayionisation Massenspektrometrie) [M+H]⁺ = 299.

### Synthese von N-(1,3-Dimethylbulyl)-N'-p-hydroxyphenyl-p-phenylendiamin (erfindungsgemäße Verbindung gemäß Formel I)) gemäß Schritt e):

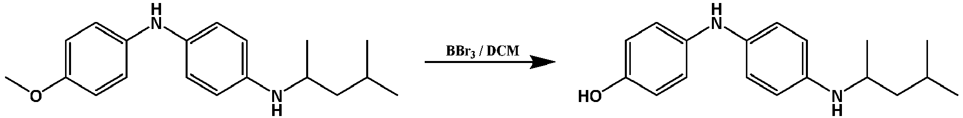

Unter Argon wurden 5.70 g (19.1 mmol, 1 Äq) N-(1,3-Dimethylbutyl)-N'-p-methoxyphenyl-p-phenylendiamin in 20.0 mL trockenem Dichlormethan (DCM) gelöst und auf 0°C gekühlt. Anschließend wurden 5.52 mL (57.3 mmol, 5 Äq) Bortribromid in 15 mL trockenem DCM gelöst und vorsichtig zugtropft. Es wurde über Nacht rühren gelassen und die Reaktion unter Schutzgas und Eiskühlung durch Zugabe von ges. NaHCO₃- Lösung (Lsg.) abgebrochen. Zusätzlich wurden noch 50 mL einer 3:1-Mischung Essigester/Isopropanol zugegeben. Die organische Phase wurde mit gesättigter (ges.) NaHCO₃-Lsg sowie ges. NaCl-Lsg. gewaschen und über MgSO₄ getrocknet. Nach erfolgter Filtration wurde das Lösungsmittel bis zur Trockne eingeengt und im Vakuum getrocknet. Hellvioletter bis blauer Feststoff; Ausbeute 4.8 g (88 % d. Th.).

¹H-NMR (500 MHz, DMSO-*d6*) δ = 8.63 (s, 1H), 6.95 (s, 1H), 6.76 (d, *J* = 8.7 Hz, 2H), 6.71 (d, *J* = 8.8 Hz, 2H), 6.59 (d, *J* = 8.7 Hz, 2H), 6.51 - 6.44 (m, 2H), 4.71 (d, *J* = 8.2 Hz, 1H), 3.40-3.31 (d, *J* = 6.9 Hz, 1H), 1.73 (dp, *J* = 13.4, 6.7 Hz, 1H), 1.49 - 1.37 (m, 1H), 1.23 - 1.15 (m, 1H), 1.05 (d, *J* = 6.1 Hz, 3H), 0.91 (d, *J* = 6.6 Hz, 3H), 0.87 (d, *J* = 6.6 Hz, 3H).

¹³C-NMR (126 MHz, DMSO-*d6*) δ = 149.8, 142.5, 138.1, 133.9, 119.9, 117.0, 115.6, 113.3, 46.1, 45.9, 26.3, 24.5, 22.8, 22.6, 20.8 (46.1 und 45.9 ist die Aufspaltung als Folge von Isomeren).

ESI-MS [M+H]⁺ = 285.

Die Zwischenverbindung gemäß Formel C) wurde in einer alternativen Variante der Umsetzung gemäß Schritt c) auf folgende Weise hergestellt:

### X2): Synthese von N-(1,3-Dimethylbutyl)-N'-p-methoxyphenyl-p-phenylendiamin:

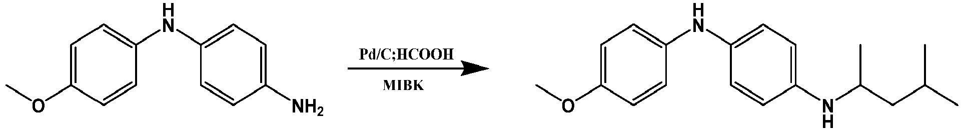

Unter Schutzgas wurden 0.40 g Palladium auf Kohle (5%) in 20 mL trockenem Ethanol suspendiert und 1.00 g (4.67 mmol, 1 Äq) 4-(4-Methoxyphenylamino)anilin, 0.88 mL (23.3 mmol, 5 Äq) Ameisensäure (23.3 mmol, 5 Äq) und 1.17 mL (9.33 mmol, 2 Äq) Methylisobutylketon nacheinander zugegeben. Es wurde für 4 Stunden unter Rückfluss erhitzt, von den Feststoffen filtriert sowie das Lösungsmittel im Vakuum entfernt. Der Rückstand wurde an Kieselgel aufgereinigt (Cyclohexan/Essigester 100:0 → 80:20). Oranges Öl; Ausbeute 1.0 g (73 % d. Th.).

Die Spektren sind identisch zur ersten Durchführung (X1).

Die Durchführung des Verfahrensschrittes c) nach X1) ist aufgrund der besseren Reproduzierbarkeit und dabei weniger anfallenden Nebenprodukten gegenüber der nach X2) (Formiat als Nebenprodukt aus der Reaktion von Amin und Ameisensäure) bevorzugt.

Ferner wurde die erfindungsgemäße Verbindung durch eine alternative Syntheseroute folgendermaßen hergestellt:

### Synthese von N-p-Hydroxyphenvl-p-phenylendiamin (Verbindung gemäß Formel C1) gemäß Schritt c1):

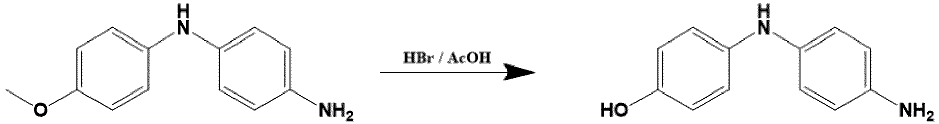

Unter Schutzgas wurden 20 mL entgaste Essigsäure sowie 10 mL Bromwasserstoffsäure vorgelegt und darin 1 g (4.67 mmol, 1 Äq) 4-(4-Methoxyphenylamino)anilin gelöst und für 24 Stunden bei 120°C gerührt. Nachdem die Lösung auf RT gebracht wurde, wurde mittels ges. NaHCO₃-Lsg. neutral gestellt und mit einer 3:1-Mischung Essigester/Isopropanol extrahiert. Das Lösungsmittel wurde entfernt und der Rückstand an Kieselgel aufgereinigt (Cyclohexan/Essigester 1:1). Brauner Feststoff; Ausbeute 0.7 g (75 % d. Th.).

¹H-NMR (500 MHz, DMSO-*d6*) δ = 8.64 (s, 1H), 6.94 (s, 1H), 6.73 - 6.68 (m, 4H), 6.59 (d, *J* = 8.8 Hz, 2H), 6.48 (d, *J* = 8.6 Hz, 2H), 4.56 (s, 2H).

### Synthese von N-(1,3-Dimethylbulyl)-N'-p-hydroxyphenyl-p-phenylendiamin (erfindungsgemäße Verbindung gemäß Formel I)) gemäß Schritt e1):

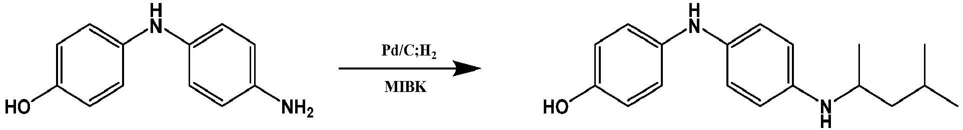

In einen Edelstahlautoklaven, der mit einem Tefloninliner bestückt wurde, wurden 0.31 g (1.55 mmol, 1 Äq) N-p-hydroxyphenyl-p-phenylendiamin, 0.66 g Palladium auf Kohle (5%) (0.2 g auf 4.67 mmol Substrat) und 20.0 mL Methylisobutylketon eingewogen. Anschließend wurde 20 bar Wasserstoff aufgedrückt und bei 60°C für 10 Stunden rühren gelassen. Nach Beendigung der Reaktion wurde der überschüssige Wasserstoff abgeblasen und die Suspension über Kieselgur (Celite^{®}) filtriert sowie mit Ethanol nachgewaschen. Das Filtrat wurde bis zur Trockne eingeengt und im Vakuum getrocknet. Das Rohprodukt enthält nach NMR-Analyse 70% des Zielmoleküls.

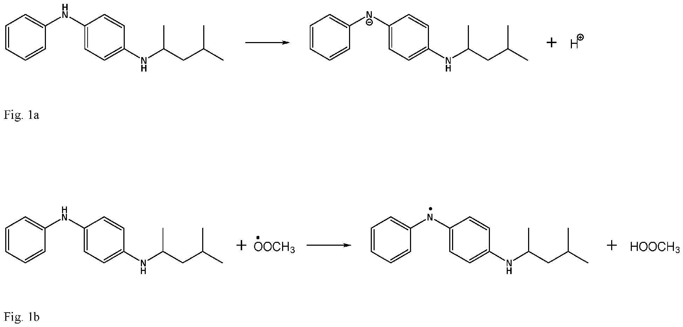

Die erfindungsgemäße Verbindung gemäß Formel I) weist ggü. 6PPD eine erhöhte Reaktivität, auf. Dies ließ sich beispielsweise anhand der Berechnung von Bindungsdissoziationsenergien (BDE), sowie der freien Aktivierungsenthalpie Δ_{R}G^{≠} bzw. der freien Standardreaktionsenthalpie Δ_{R}G° für die Reaktion mit einem Methylperoxidradikal nachvollziehen. Die Werte sind in Tabelle 1 angegeben. In Fig.1a und 1b sind die Spaltmechanismen dargestellt, auf die sich die Werte beziehen.

**Tabelle 1**

| **Molekül** | **BDE [kJ/mol]** | **Δ_{R}G° [kJ/mol]** | **Δ_{R}G^{≠} [kJ/mol]** |
|---|---|---|---|
| 6PPD | 313 | -25,6 | 19,1 |
| Formel I) | 305 | -28,8 | 16,0 |

Wie Tabelle 1 zeigt, ergeben sich für die erfindungsgemäße Verbindung gemäß Formel I) eine niedrigere Bindungsdissoziationsenergie und niedrigere freie Enthalpien.

Mit der Verbindung gemäß Formel I) kann somit eine verbesserte Schutzwirkung bei den genannten Anwendungsmöglichkeiten erzielt werden.

Für die Anwendung in einer Kautschukmischung für Fahrzeugreifen wird die erfindungsgemäße Verbindung gemäß Formel I) beispielsweise anstelle der im Stand der Technik bekannten Alterungsschutzmittel, wie 6PPD, 7PPD oder IPPD usw., auf dem Fachmann bekannte Weise in einer der Mischstufen bei der Herstellung der Kautschukmischung zugegeben.

## Patentansprüche

1. Verbindung gemäß Formel I):

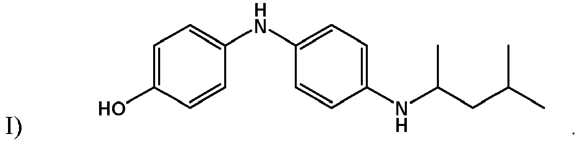

2. Kautschukmischung enthaltend die Verbindung gemäß Formel I) nach Anspruch 1.

3. Kautschukmischung nach Anspruch 2, **dadurch gekennzeichnet, dass** sie wenigstens einen Dienkautschuk enthält.

4. Kautschukmischung nach Anspruch 3, **dadurch gekennzeichnet, dass** sie wenigstens einen Dienkautschuk enthält, der ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

5. Fahrzeugreifen, der die Kautschukmischung nach einem der Ansprüche 2 bis 4 in wenigstens einem Bauteil aufweist.

6. Fahrzeugreifen nach Anspruch 5, **dadurch gekennzeichnet, dass** er wenigstens eine Kautschukmischung nach einem der Ansprüche 2 bis 4 in wenigstens einem äußeren Bauteil aufweist, wobei das äußere Bauteil bevorzugt ein Laufstreifen, eine Seitenwand und/oder ein Hornprofil ist.

7. Verfahren zur Herstellung der Verbindung gemäß Formel I) nach Anspruch 1 umfassend wenigstens die folgenden Verfahrensschritte:
a) Bereitstellung der Substanz gemäß Formel A) 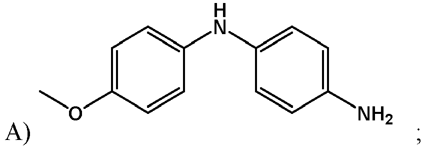
b) Bereitstellung von Methylisobutylketon (MIBK) und Wasserstoff oder von Methylisobutylketon (MIBK) und Ameisensäure ;
c) Umsetzung der Substanz gemäß Schritt a) mit den Substanzen gemäß Schritt b) zu der Substanz gemäß Formel C) 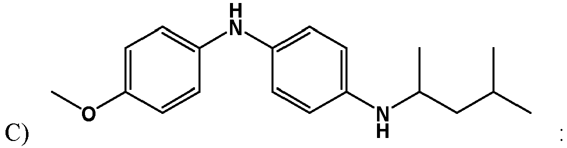
d) Bereitstellung einer Lewis-Säure, wie insbesondere Bortribromid (BBr₃), Aluminiumtrichlorid (AlCl₃), Aluminiumtribromid (AlBr₃), Bortrifluoriddiethyletherat (BF₃*OEt₂) oder Zinntetrachlorid (SnCl₄), oder einer Brönsted-Säure (Brønsted-Säure), wie insbesondere Iodwasserstoff (HI) oder Bromwasserstoff (HBr);
e) Umsetzung der Substanz gemäß Formel C) mit der Substanz aus Schritt d) zu der Substanz gemäß Formel I)

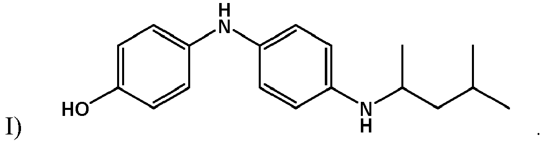

8. Verfahren zur Herstellung der Verbindung gemäß Formel I) nach Anspruch 1 umfassend wenigstens die folgenden Verfahrensschritte:
a1) Bereitstellung der Substanz gemäß Formel A) 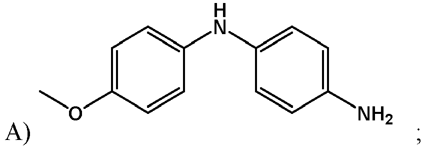
b1) Bereitstellung einer Lewis-Säure, wie insbesondere Bortribromid (BBr₃), Aluminiumtrichlorid (AlCl₃), Aluminiumtribromid (AlBr₃), Bortrifluoriddiethyletherat (BF₃*OEt₂) oder Zinntetrachlorid (SnCl₄), oder einer Brönsted-Säure (Brønsted-Säure), wie insbesondere Iodwasserstoff (HI) oder Bromwasserstoff (HBr);
c1) Umsetzung der Substanz gemäß Schritt a1) mit der Substanz aus Schritt
b1) zu der Substanz gemäß Formel C1) 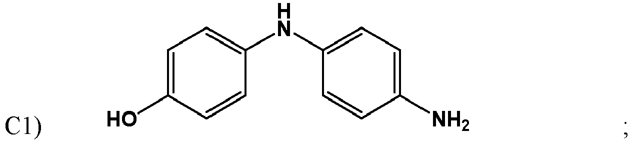
d1) Bereitstellung von Methylisobutylketon (MIBK) und Wasserstoff;
e1) Umsetzung der Substanz gemäß Formel C1) mit Methylisobutylketon (MIBK)
aus Schritt d1) zu der Substanz gemäß Formel I)

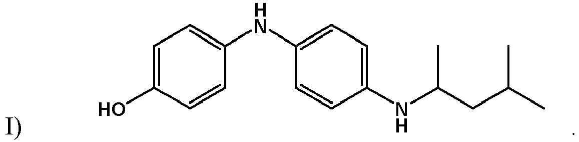

9. Verwendung der Verbindung gemäß Formel I) nach Anspruch 1 als Alterungsschutzmittel und/oder Ozonschutzmittel insbesondere in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

10. Verwendung der Verbindung gemäß Formel I) nach Anspruch 1 zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon.

11. Verwendung der Verbindung gemäß Formel I) nach Anspruch 1 in Ölen, Schmierstoffen, wie insbesondere Treib- oder Betriebsstoffen für Motoren.

12. Verwendung der Verbindung gemäß Formel I) nach Anspruch 1 als Farbstoff als Farbstoff in Fasern und/oder Polymeren und/oder Papier und/oder in (Streich-) Farben und Lacken.

## Claims

1. Compound of formula I):

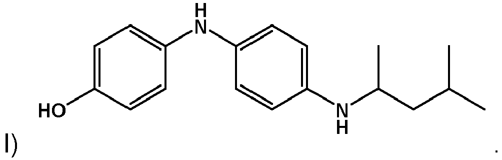

2. Rubber mixture containing the compound of formula I) according to Claim 1.

3. Rubber mixture according to Claim 2, **characterized in that** it contains at least one diene rubber.

4. Rubber mixture according to Claim 3, **characterized in that** it contains at least one diene rubber selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR) and halobutyl rubber.

5. Vehicle tyre comprising the rubber mixture according to any of Claims 2 to 4 in at least one component.

6. Vehicle tyre according to Claim 5, **characterized in that** it comprises at least one rubber mixture according to any of Claims 2 to 4 in at least one outer component, wherein the outer component is preferably a tread, a sidewall and/or a flange profile.

7. Process for producing the compound of formula I) according to Claim 1 comprising at least the process steps of:
a) providing the substance of formula A) 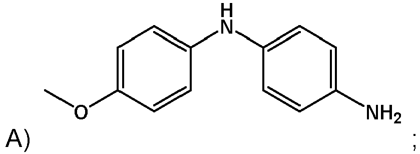
b) providing methyl isobutyl ketone (MIBK) and hydrogen or methyl isobutyl ketone (MIBK) and formic acid;
c) reacting the substance of step a) with the substances of step b) to afford the substance of formula C) 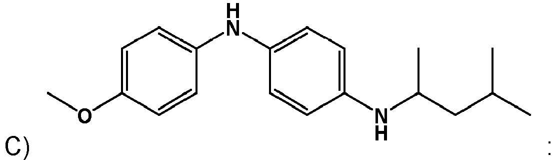
d) providing a Lewis acid, such as especially boron tribromide (BBr₃), aluminium trichloride (AlCl₃), aluminium tribromide (AlBr₃), boron trifluoride diethyl etherate (BF₃*OEt₂) or tin tetrachloride (SnCl₄), or a Bronsted acid, such as especially hydrogen iodide (HI) or hydrogen bromide (HBr);
e) reacting the substance of formula C) with the substance from step d) to afford the substance of formula I)

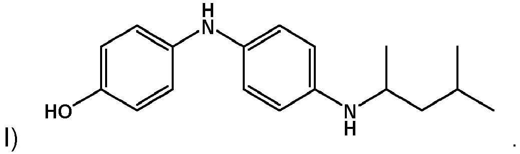

8. Process for producing the compound of formula I) according to Claim 1 comprising at least the process steps of:
a1) providing the substance of formula A) 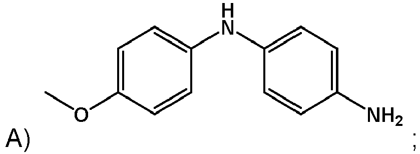
b1) providing a Lewis acid, such as especially boron tribromide (BBr₃), aluminium trichloride (AlCl₃), aluminium tribromide (AlBr₃), boron trifluoride diethyl etherate (BF₃*OEt₂) or tin tetrachloride (SnCl₄), or a Bronsted acid, such as especially hydrogen iodide (HI) or hydrogen bromide (HBr);
c1) reacting the substance of step a1) with the substance from step b1) to afford the substance of formula C1) 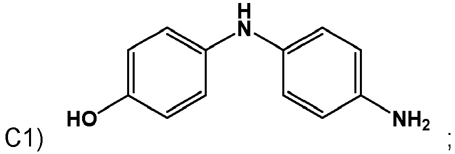
d1) providing methyl isobutyl ketone (MIBK) and hydrogen;
e1) reacting the substance of formula C1) with methyl isobutyl ketone (MIBK) from step d1) to afford the substance of formula I)

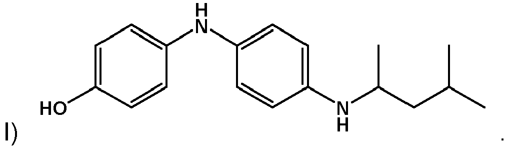

9. Use of the compound of formula I) according to Claim 1 as an ageing stabilizer and/or antiozonant in particular in vehicle tyres and/or technical rubber articles, such as in particular an air spring, bellows, conveyor belt, strap, drive belt, hose, rubber band, profile, a seal, a membrane, tactile sensors for medical applications or robotics applications, or a shoe sole or parts thereof and/or oils and/or lubricants.

10. Use of a compound of formula I) according to Claim 1 for producing a rubber article, in particular an air spring, bellows, conveyor belt, strap, drive belt, hose, rubber band, profile, a seal, a membrane, tactile sensors for medical applications or robotics applications, or a shoe sole or parts thereof.

11. Use of the compound of formula I) according to Claim 1 in oils, lubricants, such as especially fuels or fluids for engines.

12. Use of the compound of formula I) according to Claim 1 as a dye in fibres and/or polymers and/or paper and/or in (decorating) paints and coatings.

## Revendications

1. Composé selon la formule (I) :

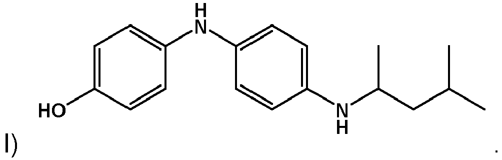

2. Mélange de caoutchouc contenant le composé selon la formule I) selon la revendication 1.

3. Mélange de caoutchouc selon la revendication 2, **caractérisé en ce qu'**il contient au moins un caoutchouc diénique.

4. Mélange de caoutchouc selon la revendication 3, **caractérisé en ce qu'**il contient au moins un caoutchouc diénique, qui est choisi dans le groupe constitué par le polyisoprène naturel (NR), le polyisoprène synthétique (IR), le caoutchouc de butadiène (BR), le caoutchouc de styrène-butadiène polymérisé en solution (SSBR), le caoutchouc de styrène-butadiène polymérisé en émulsion (ESBR), le caoutchouc butyle (IIR) et le caoutchouc halogénobutyle.

5. Pneumatique pour véhicule qui présente le mélange de caoutchouc selon l'une des revendications 2 à 4 dans au moins un composant.

6. Pneumatique pour véhicule selon la revendication 5, **caractérisé en ce qu'**il présente au moins un mélange de caoutchouc selon l'une des revendications 2 à 4 dans au moins un composant externe, le composant externe étant de préférence une bande de roulement, un flanc latéral et/ou un profilé de protection.

7. Procédé de préparation du composé selon la formule I) selon la revendication 1, comprenant au moins les étapes de procédé suivantes :
a) mise à disposition de la substance selon la formule A) 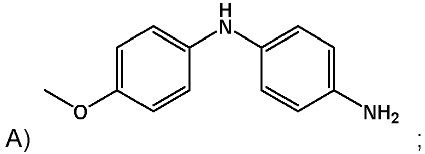
b) mise à disposition de méthylisobutylcétone (MIBK) et d'hydrogène ou de méthylisobutylcétone (MIBK) et d'acide formique ;
c) transformation de la substance selon l'étape a) avec les substances selon l'étape b) en substance selon la formule C) 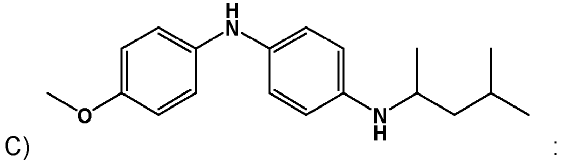
d) mise à disposition d'un acide de Lewis, tel qu'en particulier le tribromure de bore (BBr₃), le trichlorure d'aluminium (AlCl₃), le tribromure d'aluminium (AlBr₃), le diéthyléthérate de trifluorure de bore (BF₃*OEt₂) ou le tétrachlorure d'étain (SnCl₄), ou d'un acide de Brönsted (acide de Bronsted), tel qu'en particulier l'acide iodhydrique (Hl) ou l'acide bromhydrique (HBr) ;
c) transformation de la substance selon la formule C) avec la substance de l'étape d) en substance selon la formule I)

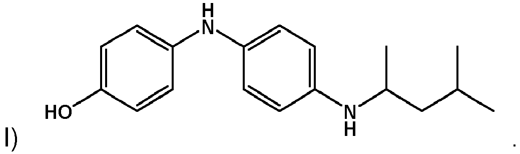

8. Procédé de préparation du composé selon la formule I) selon la revendication 1, comprenant au moins les étapes de procédé suivantes :
a1) mise à disposition de la substance selon la formule A) 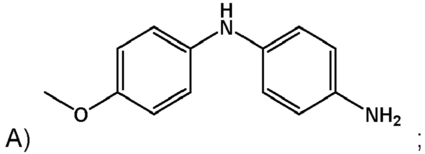
b1) mise à disposition d'un acide de Lewis, tel qu'en particulier le tribromure de bore (BBr₃), le trichlorure d'aluminium (AlCl₃), le tribromure d'aluminium (AlBr₃), le diéthyléthérate de trifluorure de bore (BF₃*OEt₂) ou le tétrachlorure d'étain (SnCl₄), ou d'un acide de Brönsted (acide de Bronsted), tel qu'en particulier l'acide iodhydrique (Hl) ou l'acide bromhydrique (HBr) ;
1c) transformation de la substance selon la formule a1) avec la substance de l'étape b1) en substance selon la formule C1) 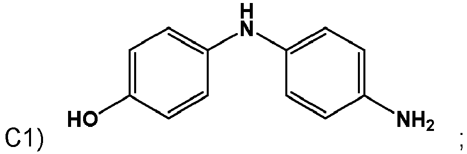
d1) mise à disposition de méthylisobutylcétone (MIBK) et d'hydrogène ;
e1) transformation de la substance selon la formule C1) avec la méthylisobutylcétone (MIBK) de l'étape d1) en substance selon la formule I)

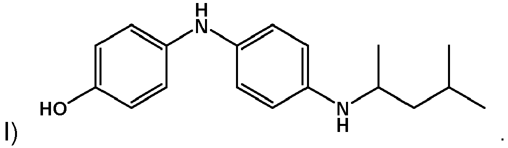

9. Utilisation du composé selon la formule I) selon la revendication 1 comme agent de protection contre le vieillissement et/ou contre l'ozone, en particulier dans des pneumatiques de véhicule et/ou des articles techniques en caoutchouc, tels qu'en particulier un ressort pneumatique, un soufflet, une bande transporteuse, une sangle, une courroie, un tuyau, une bande en caoutchouc, un profilé, un joint, une membrane, des capteurs tactiles pour des applications médicales ou robotiques ou une semelle de chaussure ou des parties de celle-ci et/ou des huiles et/ou des lubrifiants.

10. Utilisation du composé selon la formule I) selon la revendication 1 pour la fabrication d'un article en caoutchouc, en particulier d'un ressort pneumatique, d'un soufflet, d'une bande transporteuse, d'une sangle, d'une courroie, d'un tuyau, d'une bande en caoutchouc, d'un profilé, d'un joint, d'une membrane, de capteurs tactiles pour des applications médicales ou robotiques ou d'une semelle de chaussure ou des parties de celle-ci.

11. Utilisation du composé selon la formule I) selon la revendication 1 dans des huiles, des lubrifiants, tels qu'en particulier des carburants ou lubrifiants pour des moteurs.

12. Utilisation du composé selon la formule I) selon la revendication 1 comme colorant dans des fibres et/ou des polymères et/ou du papier et/ou des peintures et des laques (d'enduction).
